# EUROPEAN PATENT SPECIFICATION

(11) **EP 0 365 194 B1**
(45) Date of publication and mention of the grant of the patent: **02.08.1995**
(21) Application number: 89310319.2
(22) Date of filing: 09.10.1989
(51) Int. Cl.: C07D 209/34, A61K 31/40

(54) **Prodrugs of antiinflammatory 3-acyl-2-oxindole-1-carboxamides**
Prodrogen von antiinflammatorischen 3-Acyl-2-oxindol-1-carboxamiden
Prodrogues de 3-aryl-2-oxindole-1-carboxamide antiinflammatoires

(30) Priority: 18.10.1988 US 3658
(43) Date of publication of application: 25.04.1990
(73) Proprietor: PFIZER INC., New York, N.Y. 10017 (US)
(72) Inventor: Crawford, Thomas Charles, Glastonbury Connecticut (US); Reiter, Lawrence Alan, Mystic Connecticut (US)
(74) Representative: Wood, David John

(56) References cited:
- EP-A- 0 393 936
- WO-A-88/05656
- US-A- 4 556 672
- CHEMICAL ABSTRACTS, vol. 78, 1973, page 363, abstract no. 136057s, Columbus, Ohio, US; & JP-A-73 74 667
- CHEMICAL ABSTRACTS, vol. 110, 1989, page 39, abstract no. 185555r, Columbus, Ohio, US; E. MOILANEN et al.: "CP-66,248, a new anti-inflammatory agent, is a potent inhibitor of leukotriene B4 and prostanoid synthesis in human polymorphonuclear leukocytes in vitro"

## Description

The present invention is concerned with antiinflammatory agents and, in particular, with enol esters and ether prodrugs of 3-acyl-2-oxindole-1-carboxamides, a class of known nonsteroidal antiinflammatory agents.

The use of oxindoles as antiinflammatory agents was first reported in U.S. 3,634,453, and consisted of 1-substituted-2-oxindole-3-carboxamides. Recently, a series of 3-acyl-2-oxindole-1-carboxamides was disclosed in U.S. 4,556,672 to be inhibitors of the cyclooxygenase (CO) and lipoxygenase (LO) enzymes and to be useful as analgesic and antiinflammatory agents in mammalian subjects.

The present invention provides antiinflammatory ether and ester prodrugs of the formula 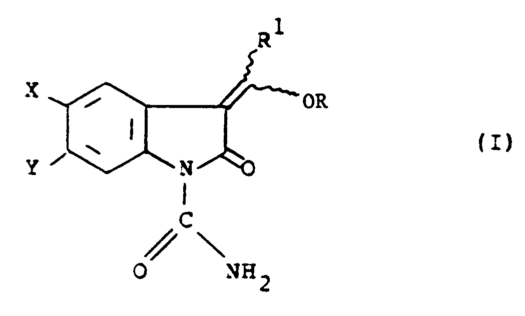
wherein X and Y are each hydrogen, fluoro or chloro; R¹ is 2-thienyl or benzyl; and R is alkanoyl of two to ten carbon atoms, cycloalkylcarbonyl of five to seven carbon atoms, phenylalkanoyl of seven to ten carbon atoms, chorobenzoyl, methoxybenzoy, thenoyl, omega-alkoxycarbonylalkanoyl said alkoxy having one to three carbon atoms and said alkanoyl having three to five carbon atoms; alkoxy carbonyl of two to ten carbon atoms; phenoxycarbonyl; 1-(acyloxy)alkyl said acyl having one to four carbon atoms and said alkyl having one to four carbon atoms; 1-(alkoxycarbonyloxy)alkyl said alkoxy having two to five carbon atoms and said alkyl having one to four carbon atoms; alkyl of one to three carbon atoms; alkylsulfonyl of one to three carbon atoms; methylphonylsulfonyl or dialkylphosphonate said alkyl each of one to three carbon atoms.

Particularly preferred are compound of formula (I) where R¹ is 2-thienyl, X is chloro, Y is hydrogen, especially those in which R is alkanoyl of two to ten carbon atoms. Preferred within this group are compounds where R is acetyl, propionyl and i-butyryl.

A second preferred group of compounds of formula (I) are those where R¹ is 2-thienyl, X is chloro, Y is hydrogen and R is phenylalkanoyl of seven to ten carbon atoms. Especially preferred within this group is the compound where R is phenylacetyl.

A third preferred group of compounds are those of formula (I) where R¹ is 2-thienyl, X is chloro, Y is hydrogen and R is omega alkoxycarbonylalkanoyl, said alkoxy having one to three carbon atoms and said alkanoyl having three to five carbon atoms. Especially preferred within this group is the compound where R is omega ethoxycarbonylpropionyl.

A fourth group of preferred compounds of formula (I) are those where R¹ is 2-thienyl, X is chloro, Y is hydrogen and R is alkoxycarbonyl of two to ten carbon atoms. Especially preferred within this group are compounds where R is methoxycarbonyl, ethoxycarbonyl and n-hexoxycarbonyl.

A fifth group of preferred compounds of formula (I) are those where R¹ is 2-thienyl, X is chloro, Y is hydrogen and R is 1-(alkoxycarbonyloxy)alkyl said alkoxy having two to five carbon atoms and said alkyl having one to four carbon atoms. Especially preferred within this group is the compound where R is 1-(ethoxycarbonyloxy)ethyl.

A sixth group of preferred compounds of formula (I) are those where R¹ is 2-thienyl, X is chloro, Y is hydrogen and R is alkylsulfonyl of one to three carbon atoms. Especially preferred within this group is the compound where R is methylsulfonyl.

A seventh group of preferred compounds of formula (I) are those where R¹ is 2-thienyl, X is fluoro, Y is chloro, especially those where R is alkanoyl of two to ten carbon atoms. Especially preferred within this group are the compounds where R is acetyl, propionyl and i-butyryl.

An eighth group of preferred compounds of formula (I) are those where R¹ is 2-thienyl, X is fluoro, Y is chloro and R is alkoxycarbonyl of two to ten carbon atoms. Especially preferred within this group are the compounds where R is methoxycarbonyl, ethoxycarbonyl and n-hexoxycarbonyl.

The ninth group of preferred compounds of formula (I) are those where R¹ is benzyl, X is hydrogen, Y is fluoro, especially when R is alkanoyl having two to ten carbon atoms. Especially preferred within this group is the compound where R is acetyl.

The tenth group of preferred compounds of formula (I) are those where R¹ is benzyl, X is hydrogen, Y is fluoro and R is alkoxycarbonyl of two to ten carbon atoms. Especially preferred with this group is the compound where R is methoxycarbonyl.

The present invention also comprises a pharmaceutical composition comprising a compound of formula (I) as defined above and a pharmaceutically acceptable diluent or carrier. It also relates to such a compound for use as a medicament, and use of the compound for manufacture of a medicament for use as an antiinflammatory agent.

The enol ethers and esters of the present invention are not enolic acids as the parent compounds are and show reduced gastric irritation when compared to said parent compounds.

The term "prodrug" refers to compounds which are drug precursors which, following administration and absorption, release the drug in vivo via some metabolic process.

While all of the usual routes of administration are useful with the invention compounds, the preferred route of administration is oral. After gastrointestinal absorption, the present compounds are hydrolyzed in vivo, to the corresponding compounds of formula (I) where R is hydrogen, or a salt thereof. Since the prodrugs of the invention are not enolic acids, exposure of the gastrointestinal tract to the acidic parent compound is thereby minimized. Further, since gastrointestinal complications have been noted as a major adverse reaction of acid non-steroidal antiinflammatory drugs (see e.g., DelFavero in "Side Effects of Drugs Annual 7", Dukes and Elis, Eds. Excerpta Medica, Amsterdam, 1983, p. 104-115], the invention compounds (I) have a distinct advantage over the parent enolic compounds.

In converting the 3-acyl-2-oxindole-1-carboxamides to the compounds of formula I, the substituents on the exocyclic double bond at the 3-position can be syn, anti or a mixture of both. Thus the compounds of the structures 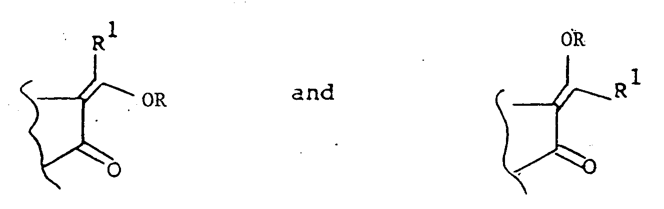
or mixtures thereof are depicted as 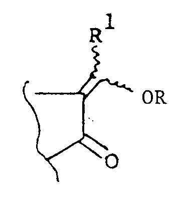
All forms of these isomers are considered part of the present invention.

There are two methods employed in the synthesis of the compounds of the present invention; the first method comprises treating a solution of the appropriate 3-acyl-2-oxindole-1-carboxamide and an equimolar amount of tertiary amine such as triethylamine in a reaction-inert solvent such as chloroform, at 0°C with an equimolar amount, plus a slight excess of the requisite acid chloride, chloroformate, oxonium salt or alkylating agent. The reaction is allowed to warm to room temperature and remain for about 2-3 hours. If the starting oxindole is not completely reacted the mixture is cooled to 0°C, additional acylating or alkylating agent is added and the process repeated until all the starting oxindole is consumed.

The product is isolated from the reaction solvent after it has been washed with 1N hydrochloric acid followed by a saturated sodium bicarbonate solution extraction. The residual product, remaining after the solvent has been removed in vacuo, is purified by recrystallization or chromatography.

The second procedure, useful in the preparation of the products of the present invention, consists of contacting, in an anhydrous reaction-inert solvent such as acetone, the appropriate 3-acyl-2-oxindole-1-carboxamide a three-four-fold molar excess of the requisite alpha-chloroalkylcarbonate, a five fold molar excess of sodium iodide and a two fold molar excess of anydrous potassium carbonate and heating said reaction mixture at reflux for 16 hours.

The reaction mixture is diluted with water and the product extracted with a water-immiscible solvent, such as diethyl ether or chloroform. Concentration of the solvent containing the product provides the crude material, which can be purified by recrystallization and/or chromatography.

The 3-acyl-2-oxindole-1-carboxamides required as starting materials are available by methods well known in the art, see, for example, the reference to these compounds cited above. The other starting reagents noted above are available commercially, or are prepared by well known methods.

The prodrugs of formula (I) are evaluated for their antiinflammatory and analgesic activity according to known methods such as the rat foot edema test, rat adjuvant-induced arthritis test or phenylbenzoquinone-induced writhing test in mice, as previously used in the evaluation of the parent compounds and described in the references cited above and elsewhere in the literature; see e.g., C. A. Winter, in "Progress in Drug Research" edited by E. Jucker, Birkhauser Verlag, Basel, Vol. 10, 1966, pp. 139-192.

In comparison with the parent 3-acyl-2-oxindole-1-carboxamides the novel prodrugs of formula (I) are found to have reduced ability to inhibit prostaglandin synthesis from arachidonic acid in tests carried out by a modification of the method of T. J. Carty et al., Prostaglandins, 19, 51-59 (1980). In the modified procedure cultures of rat basophilic leukemic cells (RBL-1), prepared by the method of Jakschik et al., ibid., 16, 733 (1978), are employed in place of mouse fibroblast (MC5-5) and rabbit synovial cell cultures. Thus, the invention compounds themselves are relatively inactive as antiinflammatory agents, but they give rise to an active antiinflammatory compound upon hydrolysis in vivo. Since the compounds (I) are not enolic acids and it is known that the hydrolysis takes place after the prodrug leaves the stomach, they will significantly reduce the gastric irritation caused by oral administration of the parent enolic compounds.

On a molar basis, the present prodrugs are generally dosed at the same level and frequency as the known 3-acyl-2-oxindole-1-carboxamides from which they are derived. However, the non-enolic nature of the present compounds will generally permit higher tolerated oral doses, when such higher dosage is required in the control of pain and inflammation.

The present prodrugs are also formulated in the same manner, and administered by the same routes as the known parent compounds, as described in the above cited reference. The preferred route of administration is oral, thus taking particular advantage of the non-enolic nature of the present compounds.

The present invention is illustrated by the following examples.

### EXAMPLE 1

### General Procedures

### Method A

To a slurry of a 3-acyl-2-oxindole-1-carboxamide in chloroform is added an equimolar amount of triethylamine. The resulting solution is cooled to 0°C and a slight excess of the appropriate acid chloride, chloroformate, oxonium salt or alkylating agent added. After stirring for 2 hours at 0°C and then at room temperature for 2 hours, if the 3-acyloxindole-1-carboxamide has not been consumed, then the mixture is again cooled to 0°C and additional acid chloride chloroformate or oxonium salt is added and the mixture stirred at 0°C for 2 hours and then at room temperature for 2 hours. This process may be repeated in order to ensure complete consumption of the 3-acyloxindole-1-carboxamide. Upon completion of the reaction, the mixture is filtered and the filtrate washed with 1N hydrochloric acid (2X) and saturated sodium bicarbonate solution (2X). The organic layer is dried with MgSO₄, filtered and concentrated in vacuo. The resulting product is purified by recrystallization or chromatography.

### Method B

A mixture of 3-acyl-2-oxindole-1-carboxamide, a 3-fold molar excess of the appropriate alpha-chloroalkyl- or alpha-chloro(aralkyl)carbonate, a 5-fold molar excess of sodium iodide, and a 2-fold molar excess of anhydrous potassium carbonate (dried under high vacuum at 165°C for 1 hour) in acetone (dried over molecular sieves) is refluxed for 16 hours. The cooled mixture is then diluted with water and extracted with ether. The combined ether extracts are dried with MgSO₄, filtered, and the filtrate concentrated in vacuo. The resulting crude product is purified by chromatography and/or recrystallization.

### EXAMPLE 2

Following the indicated procedure, and starting with the requisite reagents the indicated prodrugs were prepared:

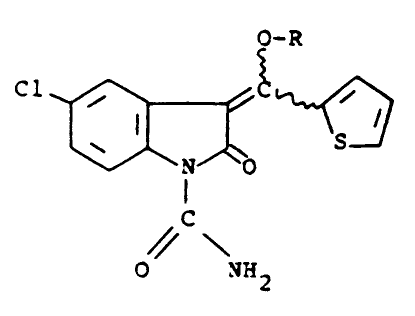

### Esters:

(R = -COCH₃) - Method A; yield 53% after recrystallization from 2-propanol; mp 173-176°C; mass spectrum m/e (relative intensity) M⁺, 362 (<1.0), 322 (4.2), 320 (11.0), 296 (1.8), 279 (18.2), 277 (44.4), 248 (10.6), 195 (77.7), 193 (100), 185 (12.3), 165 (13.4), 137 (42.8), 111 (88.2), 102 (20.0), 83 (23.9); ¹H-NMR (CDCl₃) delta 2.39, 2.53 (3H, 2s), 5.31 (1H, br s), 7.2-7.35 (2H, m), 7.48, 7.55 (1H, 2d, J=2.1Hz), 7.6-8.3 (3H, m), 8.54 (1H, br s). Anal. calcd for C₁₆H₁₁ClN₂O₄S (362.79): C, 52.97; H, 3.06; N, 7.72. Found: C, 52.91; H, 2.95; N, 7.97.

(R = -COCH₂CH₃) - Method A; yield 18% after recrystallization from 2-propanol; mp 183-185°C; mass spectrum m/e (relative intensity) M⁺, 378, 376 (<1, 1.2), 333 (0.7), 322 (6.4), 320 (18.4), 279 (17.8), 277 (44.3), 250 (2.3), 248 (9.0), 195 (27.0), 193 (100), 137 (7.8), 111 (24.1), 57 (30.0); ¹H-NMR (d₆-Me₂SO) delta 1.0-1.3 (3H, m), 2.7-3.0 (2H, q, J=7.5Hz), 6.9-7.6 (3H, m), 7.9-8.4 (5H, m). Anal. calcd for C₁₇H₁₃ClN₂O₄S (376.68): C, 54.18; H, 3.48; N, 7.43. Found: C, 53.86; H, 3.33; N, 7.28.

(R = -CO(CH₂)₅CH₃) - Method A; yield 29% after recrystallization from 2-propanol; mp 189-190°C; mass spectrum m/e (relative intensity) M⁺, 432 (0.8), 322 (13.8), 320 (37.5), 279 (34.8), 277 (87.0), 250 (5.0), 248 (17.3), 195 (26.6), 193 (100); ¹H-NMR (CDCl₃) delta 0.95 (3H, m), 1.32-1.55 (6H, m), 1.85 (2H, pentet, J=8Hz), 2.83 (2H, t, J=8Hz), 5.35 (1H, br s), 7.25 (1H, m), 7.32 (1H, m), 7.60 (1H, d), 7.72 (1H, m), 8.27 (1H, m), 8.31 (1H, d, J=10Hz), 8.62 (1H, br s). Anal. calcd for C₂₂H₂₁ClN₂O₄S (432.91): C, 58.26; H, 4.89; N, 6.47. Found: C, 58.18; H, 4.87; N, 6.42.

(R = -CO(CH₂)₈CH₃) - Method A; yield 8% after recrystallization from 2-propanol; mp 120-122°C; mass spectrum m/e (relative intensity) M⁺, 431 (<1), 322 (2.9), 320 (8.6), 279 (16.8), 277 (42.6), 262 (0.9), 260 (2.1), 250 (2.4), 248 (9.0), 195 (26.4), 193 (100), 155 (7.4), 137 (6.3), 111 (18.2); ¹H-NMR (d₆-Me₂SO) delta 0.87 (3H, s), 1.30 (13H, br s), 1.50 (1H, m), 1.65 (1H, m), 2.20 (1H, t, J=7.2Hz), 2.70 (1H, t, J=7.3Hz), 7.1-8.5 (7H, m). Anal. calcd for C₂₄H₂₇ClN₂O₄S (474.75): C, 60.68; H, 5.73; N, 5.90. Found: C, 60.64; H, 5.76; N, 5.88.

(R = -COCH(CH₃)₂) - Method A; yield 37% after recrystallization from 2-propanol; mp 189-191°C; mass spectrum m/e (relative intensity) M⁺, 392, 390 (1.2, 3.5), 322, 320 (11.7, 30.2), 279, 277 (19.2, 48.7), 250, 248 (4.6, 15.5), 195, 193 (28.7, 100); ¹H-NMR (CDCl₃) delta 1.35 (3H, d, J=8Hz, isomer A), 1.45 (3H, d, J=8Hz, isomer B), 2.93 (1H, septet, J=8Hz, isomer A), 3.05 (1H, septet, J=8Hz, isomer B), 5.38 (1H, br s, isomer A), 5.45 (1H, br s, isomer B), 7.2-7.4 (2H, m), 7.54 (1H, d), 7.7-7.8 (2H, m), 8.2-8.3 (1H, m), 8.48 (1H, br s, isomer B), 8.55 (1H, br s, isomer A) (note: isomer ratio of A to B is approximately 80:20). Exact mass calcd for C₁₈H₁₅ClN₂O₄S: 390.0449. Found: 390.0462.

(R = -COC(CH₃)₃) - Method A; yield 51% after recrystallization from 2-propanol; mp 198-200°C; mass spectrum m/e (relative intensity) M⁺, 404 (0.3), 320 (2.4), 277 (22.0), 259 (1.1), 248 (8.3), 193 (66.6), 137 (6.6), 111 (19.1), 102 (2.4), 85 (21.1), 57 (100); ¹H-NMR (CDCl₃) delta 1.39 (9H, s), 5.47 (1H, br s), 7.23 (2H, m), 7.50 (1H, d, J=2.2Hz), 7.71 (1H, dd, J=1.1, 5.0Hz), 7.77 (1H, dd, J=1.1, 3.8Hz), 8.25 (1H, d, J=8.8Hz), 8.57 (1H, br s). Anal. calcd for C₁₉H₁₇ClN₂O₄S (404.85): C, 56.36; H, 4.23; N, 6.92. Found: C, 56.05; H, 4.23; N, 6.86.

(R = -CO(cyclohexyl)) - Method A; yield 10% after recrystallization from 2-propanol; mp 189-190°C; mass spectrum m/e (relative intensity) M⁺, 430 (0.7), 381 (<1), 322 (2.3), 320 (6.5), 279 (8.0), 277 (19.8), 195 (16.3), 193 (60.0), 111 (67.1), 83 (100), 55 (25.8); ¹H-NMR (d₆-Me₂SO) delta 1.05-1.70 (11H, set of m), 6.95-7.10 (1H, m), 7.18 (1H, t, J=4.4Hz), 7.31 (1H, dd, J=2.2, 8.8Hz), 7.4 (1H, m), 7.70-8.15 (4H, set of m). Anal. calcd for C₂₁H₁₉ClN₂O₄S (429.72): C, 58.53; H, 4.44; N, 6.50. Found: C, 58.34; H, 4.32; N, 6.43.

(R = -COPh) - Method A; yield 44% after recrystallization from acetic acid; mp 228-230°C; mass spectrum m/e (relative intensity) M⁺, 424 (3.0), 381 (1.9), 277 (3.9), 260 (6.9), 248 (10.2), 232 (0.9), 212 (2.3), 185 (4.7), 168 (24.1), 140 (6.5), 105 (100), 77 (27.1); ¹H-NMR (CDCl₃) delta 5.55 (1H, br s), 7.30 (3H, m), 7.55 (3H, m), 7.65 (1H, m), 7.74 (1H, dd, J=1.0, 5.0Hz), 7.84 (1H, dd, J=1.0, 3.8Hz), 8.2-8.3 (3H, m), 8.45 (1H, br s). Anal. calcd for C₂₁H₁₃ClN₂O₄S·H₂O (442.87): C, 56.95; H, 3.41; N, 6.32. Found: C, 57.24; H, 3.08; N, 6.09.

(R = -COCH₂Ph) - Method A; yield 3% after filtration through silica gel (10:90 - methanol/chloroform) and two recrystallizations from 2-propanol; mp 207-208°C; mass spectrum m/e (relative intensity) M⁺, 438 (< 1), 395 (< 1), 322 (9.6), 320 (26.4), 279 (17.1), 277 (43.1), 195 (14.6), 193 (54.3), 91 (100); ¹H-NMR (CDCl₃/d₆-Me₂SO) delta 3.96 (2H, s), 6.20 (1H, br s), 7.02 (1H, dd, J=4.0, 5.1Hz), 7.15 (1H, dd, J=2.2, 8.8Hz), 7.3-7.4 (6H, m), 7.57 (1H, dd, J=1.2, 5.1Hz), 7.90 (1H, dd, J=1.2, 4.0Hz), 8.15 (1H, d, J=8.8Hz), 8.30 (1H, br s). Anal. calcd for C₂₂H₁₅ClN₂O₄S (438.87): C, 60.20; H, 3.45; N, 6.38. Found: C, 60.53; H, 3.38; N, 6.18.

(R = -CO(CH₂)₃Ph) - Method A; yield 13% after recrystallization from 2-propanol; mp 168-171°C; mass spectrum m/e (relative intensity) M⁺, not observed, 423 (< 1), 322 (1.0), 320 (2.9), 279 (10.2), 277 (25.7), 250 (1.5), 248 (5.6), 195 (26.7), 193 (100), 158 (0.7), 147 (72.1), 91 (99.5); ¹H-NMR (d₆-Me₂SO) delta 1.75-2.05 (2H, m), 2.22 (1H, t, J=7.4Hz), 2.55-3.00 (3H, m), 6.90-7.65 (9H, m), 7.85-8.50 (4H, m). Anal. calcd for C₂₄H₁₉ClN₂O₄S (466.75): C, 61.73; H, 4.10; N, 5.99. Found: C, 61.74; H, 4.02; N, 5.89.

(R = -CO(3-Cl-Ph) - Method A; yield 26% after recrystallization from 2-propanol/dimethylformamide; mp 210-218°C; mass spectrum m/e (relative intensity) M⁺, 460, 458 (0.5, 0.6), 279 (1.5), 277 (3.9), 250 (0.9), 248 (2.8), 195 (1.3), 193 (4.6), 141 (43.0), 139 (100), 113 (8.8), 111 (32.8); ¹H-NMR (CDCl₃) delta 5.28 (1H, br s), 7.25 (2H, m), 7.51 (2H, m), 7.62 (1H, m), 7.74 (1H, dd, J=1.1, 5.0Hz), 7.84 (1H, dd, J=1.1, 3.8Hz), 8.07 (1H, m), 8.16 (1H, m), 8.27 (1H, d, J=8.8Hz), 8.41 (1H, br s). Anal. calcd for C₂₁H₁₂Cl₂N₂O₄S (459.29): C, 54.91; H, 2.63; N, 6.10. Found: C, 54.85; H, 2.59; N, 6.04.

(R = -CO(4-MeO-Ph) - Method A; yield 11% after filtration through silica gel (5:95 - methanol/chloroform) and recrystallization from 2-propanol; mp 198-199°C; mass spectrum m/e (relative intensity) M⁺, 454 (0.3), 411 (0.3), 279 (0.3), 277 (0.6), 250 (1.3), 248 (4.2), 195 (1.1), 193 (4.0), 135 (100); ¹H-NMR (CDCl₃) delta 4.05, 4.10 (3H, 2s), 5.35, 5.46 (1H, 2 br s), 7.15 (2H, m), 7.40 (3H, m) 7.68 (1H, d, J=2.1Hz), 7.86 (1H, dd, J=1.1, 5.0Hz), 7.97 (1H, dd, J=1.1, 3.8Hz), 8.29 (1H, m), 8.41 (1H, m), 8.60, 8.77 (1H, 2 br s). Anal. calcd for C₂₂H₁₅ClN₂O₅S (454.87): C, 58.09; H, 3.32; N, 6.16. Found: C, 57.99; H, 3.22; N, 6.07.

(R = -CO(2-thienyl)) - Method A; yield 16% after being twice flash chromatographed (1st: chloroform; 2nd: 0.5:99.5 - methanol/chloroform); mp 220-222°C; mass spectrum m/e (relative intensity) M⁺, 432, 430 (0.4, 1.1), 389 (0.4), 387 (0.7), 279 (0.6), 277 (1.7), 113 (5.1), 111 (100); ¹H-NMR (d₆-Me₂SO) delta 7.3-7.5 (4H, m), 7.8-8.4 (7H, m). Exact mass calcd for C₁₉H₁₁ClN₂O₄S₂:429.9849. Found: 429.9825.

(R = -COCH₂CH₂CO₂Et) - Method A; yield 72% after recrystallization from 2-propanol; mp 132-140°C; mass spectrum m/e (relative intensity) M⁺, 448 (< 1), 405 (< 1), 360 (< 1), 305 (1.3), 303 (3.7), 279 (2.4), 277 (6.4), 195 (8.9), 193 (32.9), 129 (100), 111 (12.6), 101 (74.3); ¹H-NMR (d₆-Me₂SO) delta 1.15 (3H, m), 2.5 (2H, m), 2.55-3.2 (2H, complex set of m), 4.05 (2H, m), 6.90-7.45 (3H, complex set of m), 7.70 (1H, m), 7.85-8.45 (4H, complex set of m). Anal. calcd for C₂₀H₁₇ClN₂O₆ (448.87): C, 53.51; H, 3.82; N, 6.24. Found: C, 53.49; H, 3.70; N, 6.23.

### Carbonates:

(R = -COOCH₃) - Method A; yield 29% after recrystallization from 2-propanol/chloroform; mp 180°C softens, melts 200°C; mass spectrum m/e (relative intensity) M⁺, 380, 378 (8.5, 23.8), 337 (7.2), 335 (21.2), 293 (17.3), 291 (39.8), 250 (28.3), 248 (100), 195 (24.9), 193 (86.2), 111 (88.6); ¹H-NMR (d₆-Me₂SO) delta 3.90, 3.95 (3H, 2s), 7.3-7.5 (3H, m), 7.95-8.05 (2H, m), 8.15-8.25 (3H, m). Anal. calcd for C₁₆H₁₁ClN₂O₅S (378.22): C, 50.73; H, 2.93; N, 7.39. Found: C, 50.84; H, 2.93; N, 7.34.

(R = -COOCH₂CH₃) - Method A; yield 24% after recrystallization from 2-propanol; mp 170-175°C; mass spectrum m/e (relative intensity) M⁺, 392 (< 1.0), 320 (1.2), 305 (3.9), 277 (22.5), 259 (2.6), 248 (17.0), 193 (100), 185 (7.2), 165 (4.0), 111 (18.8); ¹H-NMR (CDCl₃) delta 1.42 (3H, t, J=7.1Hz), 4.39 (2H, q, J=7.1Hz), 5.41 (1H, br s), 7.25 (2H, m), 7.48, 7.66 (1H, 2d, J=2.1 and 2.2Hz) 7.75 (1H, m), 8.25 (2H, m), 8.57 (1H, br s). Anal. calcd for C₁₇H₁₃ClN₂O₅S (392.79): C, 51.98; H, 3.34; N, 7.13. Found: C, 51.90; H, 3.26; N, 6.93.

(R = -COOCH(CH₃)₂) - Method A; yield 37% after recrystallization from 2-propanol; mp 185-186°C; mass spectrum m/e (relative intensity) M⁺, 322, 320 (1.8, 6.5), 303 (1.6), 279 (15.2), 277 (41.3), 250 (2.2), 248 (8.5), 193 (100), 167 (1.7), 165 (2.6), 139 (1.3), 137 (4.3), 111 (12.4), 102 (8.0); ¹H-NMR (d₆-Me₂SO) delta 1.34, 1.37 (6H, 2s), 5.00 (1H, heptet, J=6.2Hz), 7.35 (1H, t, J=4.3Hz), 7.50 (1H, dd, J=8.7Hz), 7.55 (1H, m), 7.96, 8.05 (2H, 2 br s), 8.17 (2H, m), 8.25 (1H, m). Anal. calcd for C₁₈H₁₅ClN₂O₅S (406.69): C, 53.14; H, 3.72; N, 6.89. Found: C, 52.93; H, 3.65; N, 6.82.

(R = -COO(CH₂)₅CH₃) - Method A; yield 39% after recrystallization from 2-propanol; mp 110-144°C; mass spectrum m/e (relative intensity) M⁺, 448 (0.3), 405 (< 1), 322 (1.7), 320 (4.5), 279 (15.9), 277 (39.9), 195 (29.8), 193 (100), 111 (14.8); ¹H-NMR (d₆-Me₂SO) delta 0.85 (3H, br t, J=6.6Hz), 1.3 (6H, m), 1.6 (2H, m), 4.35 (2H, t, J=6.2Hz), 7.35 (1H, t, J=4.3), 7.4-7.55 (2H, m), 7.95-8.05 (2H, m), 8.15-8.25 (3H, m). Anal. calcd for C₂₁H₂₁ClN₂O₅S (448.91): C, 56.18; H, 4.72; N, 6.24. Found: C, 56.11; H, 4.60; N, 6.16.

(R = -COO(CH₂)₈CH₃) - Method A; yield 21% after recrystallization from 2-propanol; mp 118-120°C; mass spectrum m/e (relative intensity) M⁺, 490 (0.6), 368 (0.5), 322 (4.9), 320 (2.2), 279 (32.6), 277 (79.3), 250 (4.9), 248 (16.1), 195 (28.5), 193 (100); ¹H-NMR (CDCl₃) delta 0.89 (3H, m), 1.2-1.5 (12H, m), 1.76 (2H, m), 4.34 (2H, t, J=6.6Hz), 5.33 (1H, br s), 7.24 (1H, m), 7.32 (1H, dd, J=2.2, 8.8Hz), 7.68 (1H, d, J=2.1Hz), 7.74 (1H, dd, J=1.2, 5.1Hz), 8.20 (1H, dd, J=1.2, 4.0Hz), 8.29 (1H, d, J=8.8Hz), 8.58 (1H, br s). Anal. calcd for C₂₄H₂₂ClN₂O₅S (490.99): C, 58.71; H, 5.54; N, 5.71. Found: C, 58.87; H, 5.48; N, 5.64.

(R = -COOPh) - Method A; yield 8% after recrystallization from 2-propanol; mp 212-214°C; mass spectrum m/e (relative intensity) M⁺, 442, 440 (1.7, 5.7), 399 (4.4), 397 (9.7), 355 (< 1), 354 (< 1), 353 (2.9), 352 (1.7), 338 (< 1), 336 (2.5), 250 (13.4), 248 (44.3), 234 (9.7), 232 (24.0), 195 (8.1), 193 (27.7), 111 (100); ¹H-NMR (CDCl₃) delta 5.90 (1H, br s), 7.1-7.4 (7H, m), 7.74 (2H, m), 8.22 (2H, m), 8.39 (1H, br s). Anal. calcd for C₂₁H₁₃ClN₂O₅S (440.84): C, 57.21; H, 2.97; N, 6.36. Found: C, 56.99; H, 2.98; N, 6.38.

### Acetal-esters:

(R = -CH(CH₃)OCOCH₃) - Method A with the exceptions that silver nitrate (1 molar equivalent was also included in the reaction mixture and that the reaction mixture was refluxed for 24 hours; yield 9% after twice being flash chromatographed (first: 1:99 - methanol/chloroform, second: 0.5:99.5 - methanol/chloroform) and recrystallization from cyclohexane/ethyl acetate; mp 175-180°C; mass spectrum m/e (relative intensity) M⁺, 408, 406 (< 1, < 1), 364 (2.9), 362 (1.2), 322 (12.1), 320 (40.2), 279 (25.8), 277 (62.6), 195 (43.3), 193 (100); ¹H-NMR (CDCl₃) delta 1.70 (3H, d, J=5.4Hz), 1.94 (3H, s), 5.16 (1H, br, s), 6.31 (1H, q, J=5.4Hz), 7.23 (1H, dd, J=3.9, 5.2Hz), 7.27 (1H, d, J=2.2Hz), 7.52 (1H, dd, 1.2, 3.7Hz), 7.69 (1H, dd, J=1.1, 5.1Hz), 7.98 (1H, d, J=2.2Hz), 8.21 (1H, d, J=8.8Hz), 8.47 (1H, br s). Anal. calcd for C₁₈H₁₅ClN₂O₅S (406.83): C, 53.14; H, 3.72; N, 6.89. Found: C, 53.40; H, 3.61; N, 6.85.

### Acetal-carbonates:

(R = -CH(CH₃)OCOOCH₂CH₃) - Method B; yield 32% after flash chromatography (25:75 - ethyl acetate/hexane) and recrystallization from 2-propanol; mp 159-162°C; mass spectrum m/e (relative intensity) M⁺, 438, 436 (< 1.0, 1.0), 393 (< 1.0), 322 (1.9), 320 (5.3), 307 (2.0), 305 (6.3), 279 (9.9), 277 (26.9), 195 (42.5), 193 (100); ¹H-NMR (CDCl₃) delta 1.21 (3H, t, J=7.1Hz), 1.73 (3H, d, J=5.3Hz), 4.10 (2H, q, J=5.3Hz), 5.19 (1H, br s), 7.26 (2H, m), 7.52 (1H, dd, J=1.1, 3.7Hz), 7.71 (1H, dd, J=1.1, 5.0Hz), 7.97 (1H, d, J=2.2Hz), 8.22 (1H, d, J=8.7Hz), 8.47 (1H, br s). Anal. calcd for C₁₉H₁₇ClN₂O₆S (436.86): C, 52.23; H, 3.92; N, 6.41. Found: C, 52.57; H, 4.44; N, 6.03.

(R = -CH(CH₃)OCOOC(CH₃)₃) - Method B; yield 25% after flash chromatography (25:75 - ethyl acetate/hexane) and recrystallization from 2-propanol; mp 184-187°C; mass spectrum m/e (relative intensity) M⁺, 347 (0.8), 322 (4.1), 320 (2.0), 279 (16.2), 277 (53.8), 196 (11.3), 195 (34.5), 194 (13.3), 193 (100); ¹H-NMR (CDCl₃) delta 1.33 (9H, s), 1.71 (2H, d, J=5.4Hz), 5.21 (1H, br s), 6.14 (1H, q, J=5.2Hz), 7.26 (2H, m), 7.54 (1H, dd, J=1.2, 3.7Hz), 7.70 (1H, dd, J=1.2, 5.0Hz), 8.00 (1H, d, J=2.2Hz), 8.21 (1H, d, J=8.7Hz), 8.49 (1H, br s). Anal. calcd. for C₂₁H₂₁ClN₂O₆S (464.91): C, 54.25; H, 4.55; N, 6.03. Found, 54.38; H, 4.58; N, 6.09.

(R = -CH(CH₃)OCOOCH₂Ph) - Method B; yield 11% after flash chromatography (25:75 - ethyl acetate/hexane) and recrystallization from ethyl acetate/hexane; mp 140-145°C, softens 130°C; mass spectrum m/e (relative intensity) M⁺, 498 (< 1.0), 455 (< 1.0), 410 (< 1.0), 195 (10.3), 193 (32.2), 111 (62.9), 91 (100); ¹H-NMR (CDCl₃) delta 1.72 (3H, d, J=5Hz), 5.00 (1H, d, J=11Hz), 5.04 (1H, d, J=11Hz), 5.28 (1H, br s), 6.20 (1H, q, J=5Hz), 7.1-7.3 (7H, m), 7.44 (1H, m), 7.61 (1H, m), 7.93 (1H, d, J=2Hz), 8.20 (1H, d, J=9Hz), 8.40 (1H, br s). Anal. calcd for C₂₄H₁₉ClN₂O₆S (498.92): C, 57.77; H, 3.84; N, 5.62. Found: C, 57.78; H, 3.80; N, 5.59.

### Ethers:

(R = -CH₃) - Method A using trimethyloxonium tetrafluoroborate; yield 27% after recrystallization from 2-propanol; mp 186-188°C; mass spectrum m/e (relative intensity) M⁺, 335 (2.0), 334 (4.7), 291 (29.7), 277 (18.0), 260 (21.7), 248 (12.6), 193 (100), 185 (14.5), 157 (8.7), 111 (52.5); ¹H-NMR (CDCl₃) delta 3.88 (3H, s), 5.25 (1H, br s), 7.27 (3H, m), 7.69 (1H, d, J=5.7Hz), 7.88 (1H, d, J=2.2Hz), 8.21 (1H, d, J=8.7Hz), 8.49 (1H, br s). Anal. calcd for C₁₅H₁₁ClN₂O₃S (334.76): C, 53.81; H, 3.31; N, 8.37. Found: C, 54.15; H, 3.48; N, 8.10.

(R = CH₂CH₃) - Method A using triethyloxonium tetrafluoroborate; yield 22% after recrystallization from 2-propanol; mp 202-205°C; mass spectrum m/e (relative intensity) M⁺, 350, 348 (1.5, 4.6), 320 (< 1), 307 (7.3), 305 (19.6), 250 (2.2), 248 (7.4), 195 (27.0), 193 (100), 187 (1.2), 185 (4.7), 167 (1.3), 165 (3.2), 139 (2.8), 137 (8.1), 111 (24.0); ¹H-NMR (d₆-Me₂SO) delta 1.40 (3H, t, J=7.0Hz), 4.15 (2H, q, J=7.0Hz), 7.30 (1H, m), 7.35 (1H, dd, J=2.3, 8.7), 7.50 (1H, m), 7.65 (1H, s), 7.90 (1H, d, J=2.3Hz), 8.00 (1H, dd, J=1.0, 5.0Hz), 8.05 (1H, s), 8.15 (1H, d, J=8.7Hz). Anal. calcd for C₁₆H₁₃ClN₂O₃S (348.67): C, 55.09; H, 3.76; N, 8.03. Found:, 54.87; H, 3.62; N, 7.79.

### Sulfonates:

(R = -SO₂CH₃) - Method A; yield 4% after being twice filtered through silica gel (5:95 - methanol/chloroform) and recrystallization from 2-propanol; mp 180-182°C; mass spectrum m/e (relative intensity) M⁺, 400, 398 (2.8, 5.6), 357 (6.8), 355 (2.6), 261 (15.3), 259 (45.3), 250 (31.0), 248 (100), 141 (15.4), 139 (42.9), 113 (6.1), 111 (37.7); ¹H-NMR (CDCl₃) delta 3.02 (3H, s), 5.23 (1H, br s), 7.23 (1H, m), 7.37 (1H, dd, J=2.2, 8.8Hz), 7.76 (2H, m), 8.16 (1H, d, J=2.1Hz), 8.26 (1H, d, J=8.8Hz), 8.33 (1H, br s). Anal. calcd for C₁₅H₁₁ClN₂O₅S₂ (398.83): C, 45.17; H, 2.78; N, 7.03. Found: C. 45.30; H, 2.60; N, 6.78.

(R = -SO₂(4-Me-Ph) -Method A; yield 6% after recrystallization from 2-propanol; mp 200-202°C; mass spectrum m/e (relative intensity) M⁺, 474 (< 1), 433 (1.6), 431 (4.0), 404 (1.6), 402 (3.3), 250 (32.0), 248 (100), 195 (4.4), 193 (15.8), 155 (27.7), 111 (47.8), 91 (42.2); ¹H-NMR (d₆-Me₂SO) delta 2.40 (3H, s), 7.05 (1H, t, J=4.5Hz), 7.35-7.50 (4H, m), 7.65 (3H, m), 7.90 (3H, m), 8.12 (1H, d, J=8.7Hz). Anal. calcd for C₂₁H₁₅ClN₂O₅S₂ (474.78): C, 53.10; H, 3.18; N, 5.89. Found: C, 53.09; H, 3.22; N, 5.66.

### Phosphonates:

(R = -PO(OCH₂CH₃)₂) - Method A; yield 14% after being filtered through silica gel (5:95 - methanol/chloroform) and recrystallization from cyclohexane/ethyl acetate; mp 180-183°C; mass spectrum m/e (relative intensity) M⁺, 458, 456 (1.2, 3.8), 415 (7.4), 413 (17.4), 261 (31.7), 259 (100), 250 (3.1), 248 (9.2), 196 (17.1), 195 (12.5), 193 (44.6); ¹H-NMR (CDCl₃) delta 1.33 (6H, dt, J=1.2, 7.1Hz), 4.14 (4H, m), 5.23 (1H, br s), 7.32 (1H, dd, J=2.2, 8.8Hz), 7.70 (1H, dd, J=1.2, 5.0Hz), 7.83 (1H, dd, J=1.2, 3.8Hz), 8.06 (1H, d, J=2.2Hz), 8.25 (1H, d, J=8.8Hz), 8.46 (1H, br s). Anal. calcd for C₁₈H₁₈ClN₂O₆PS (456.83): C, 47.32; H, 3.97; N, 6.13. Found: C, 47.25; H, 3.83; N, 6.08.

### EXAMPLE 2

Starting with the appropriate reagents and using the indicated procedure the following compounds were prepared:

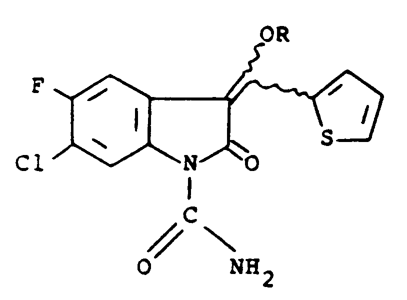

### Esters:

(R = -COCH₃) - Method A; yield 16% after recrystallization from 2-propanol; mp 190-203°C; mass spectrum m/e (relative intensity) M⁺, 382, 380 (1.6, 7.7), 340 (36.8), 338 (98.1), 297 (16.5), 295 (43.4), 279 (< 1), 277 (2.1), 268 (3.4), 266 (8.3), 256 (1.4), 254 (5.2), 213 (38.6), 211 (100), 111 (26.7); ¹H-NMR (d₆-Me₂SO) delta 1.9, 2.4 (3H, 2s), 7.09-7.40 (2H, set of m), 7.55-7.80 (1H, set of m), 7.95-8.50 (4H, set of m). Anal. calcd for C₁₆H₁₀ClFN₂O₄S (380.66): C, 50.47; H, 2.65; N, 7.36. Found: C, 50.13; H, 2.52; N, 7.19.

(R = -COCH₂CH₃) - Method A; yield 10% after filtration through silica gel (5:95 - methanol/chloroform) and recrystallization from 2-propanol; mp 182-188°C; mass spectrum m/e (relative intensity) M⁺, 396, 394 (< 1, 1.3), 340 (7.2), 338 (16.2), 297 (12.1), 295 (32.5), 268 (2.7), 266 (7.1), 213 (26.1), 211 (100), 111 (40.8), 57 (94.2); ¹H-NMR (d₆-Me₂SO) delta 1.18 (3H_{a,b}, m) 2.22 (2Hₐ, q, J=7.5Hz), 2.71 (2H_{b}, q, J=7.5Hz), 7.09-7.70 (2H_{a,b}, m), 7.95-8.48 (5H_{a,b,} m). Anal. calcd for C₁₇H₁₂ClFN₂O₄S (394.80): C, 51.71; H, 3.06; N, 7.10. Found, 51.67; H, 3.01; N, 6.97.

(R = -COCH(CH₃)₂) - Method A; yield 11% after filtration through silica gel and recrystallization from 2-propanol; mp 204-206°C; mass spectrum m/e (relative intensity) M⁺, 410, 408 (1.1, 4.1), 340 (11.5), 338 (27.2), 297 (13.2), 295 (33.6), 268 (5.6), 266 (15.0), 213 (25.8), 211 (100), 111 (36.4); ¹H-NMR (d₆-Me₂SO) delta 1.34 (6H, d, J=7.0Hz), 3.25 (1H, heptet, J=7.0Hz), 7.33 (1H, dd, J=4.0, 5.1Hz), 7.48 (1H, d, J=9.6Hz), 8.00 (1H, br s), 8.03 (1H, br s), 8.13 (1H, dd, J=1.2, 5.0Hz). Anal. calcd for C₁₈H₁₄ClFN₂O₄S (408.83): C, 52.88; H, 3.45; N, 6.85. Found: C, 52.48; H, 3.32; N, 6.86.

(R = -COCH₂Ph) - Method A; yield 22% after recrystallization from 2-propanol; mp 189-199°C; mass spectrum m/e (relative intensity) M⁺, not observed, 340 (18.9), 338 (42.1), 297 (20.7), 295 (54.4), 268 (5.8), 266 (19.6), 213 (17.9), 211 (53.7), 91 (100); ¹H-NMR (d₆-Me₂SO) delta 3.98 (2Hₐ, s), 4.02 (2H_{b}, s), 5.35 (1H, br s), 6.99-7.45 (7H, m), 7.68, 8.00 (2H, 2m), 8.42 (1H, dd, J=5.1, 6.9Hz), 8.50 (1H, br s). Anal. calcd for C₂₂H₁₄ClFN₂O₄S (456.86); C, 57.83; H, 3.09; N, 6.13. Found: C, 57.53; H, 2.98; N, 6.15.

(R = -COCH₂CH₂COOEt) - Method A; yield 26% after recrystallization from 2-propanol; mp 153-155°C; mass spectrum m/e (relative intensity) M⁺, not observed, 321 (3.1), 295 (3.1), 266 (4.5), 213 (8.8), 211 (23.4), 155 (5.2), 129 (100), 111 (12.4), 101 (75.0), 91 (2.7); ¹H-NMR (d₆-Me₂SO) delta 1.12 (3H, 2t, J=7.1Hz), 2.5-3.5 (4H, complex set of m), 4.05 (2H, 2q, J=7.3Hz), 7.15-7.40 (2H, complex set of m), 7.70 (1H, m), 7.95-8.43 (4H, complex set of m). Anal. calcd for C₂₀H₁₆ClFN₂O₆S (466.70): C, 51.45; H, 3.45; N, 6.00. Found: C, 51.28; H, 3.26; N, 5.99.

### Carbonates:

(R = -COOCH₃) - Method A; yield 25% after recrystallization from 2-propanol; mp 203-205°C; mass spectrum m/e (relative intensity) M⁺, 398, 396 (7.5, 24.5), 355 (4.5), 353 (10.6), 311 (23.8), 309 (49.1), 280 (26.3) 278 (27.9), 268 (30.5), 266 (100), 252 (3.5), 250 (6.9), 240 (3.4), 238 (7.2), 213 (25.2), 211 (56.9), 203 (29.4), 197 (5.6), 182 (6.8), 169 (6.1), 157 (4.5), 155 (12.4), 142 (2.1), 111 (45.4), 97 (5.3), 83 (5.5); ¹H-NMR (d₆-Me₂SO) delta 3.89, 3.95 (3H, 2s), 7.38 (2H, m), 8.00 (3H, m), 8.19 (1H, m), 8.29 (1H, t, J=6.7Hz). Anal. calcd for C₁₆H₁₀ClFN₂O₅S (396.71): C, 48.43; H, 2.54; N, 7.06. Found: C, 48.41; H, 2.47; N, 6.95.

(R = -COOCH₂CH₃) - Method A; yield 57% after recrystallization from 2-propanol; mp 164-166°C; mass spectrum m/e (relative intensity) M⁺, 410 (1.4), 325 (1.8), 323 (5.8), 297 (8.0), 295 (20.5), 268 (6.1), 266 (13.7), 213 (37.6), 211 (100), 203 (7.9), 155 (7.5), 111 (21.0); ¹H-NMR (d₆-Me₂SO) delta 1.30 (3H, t, J=7.1Hz), 4.32 (2H, q, J=7.1Hz), 7.35 (2H, m), 8.0 (3H, m), 8.20-8.35 (2H, m). Anal. calcd for C₁₇H₁₂ClFN₂O₅S (410.67): C, 49.70; H, 2.94; N, 6.82. Found: C, 49.76; H, 2.85; N, 6.77.

(R = -COO(CH₂)₅CH₃) - Method A; yield 85% after recrystallization from 2-propanol; mp 128-135°C; mass spectrum m/e (relative intensity) M⁺, 468, 466 (0.3, 0.7), 425 (0.3), 424 (0.3), 423 (1.1), 340 (7.0), 338 (14.1), 297 (28.5), 295 (74.5), 213 (34.3), 211 (100); ¹H-NMR (CDCl₃) delta 0.85-0.92 (3H, m), 1.22-1.48 (6H, m), 1.72 (2H, pentet, J=9Hz), 4.31 (2H_{a,b}, t), 5.40 (1H_{a,b}, br s), 7.21 (1H_{a,b}, m), 7.30 (1Hₐ, d, J=9Hz), 7.47 (1H_{b,d}, J=9Hz), 7.77 (2Hₐ, 1H_{b}, m), 8.19 (1H_{b}, m), 8.42 (1Hₐ, d, J=8Hz), 8.46 (1H_{b}, d, J=8Hz), 8.49 (1Hₐ, br s), 8.52 (1H_{b}, br s). Anal. calcd for C₂₁H₂₀ClFN₂O₅S (466.91): C, 54.02; H, 4.32; N, 6.00. Found: C, 53.93; H, 4.26; N, 6.02.

### Sulfonates:

(R = -SO₂CH₃) - Method A; yield 9% after filtration through silica gel and recrystallization from cyclohexane/ethyl acetate; mp 180-185°C; mass spectrum m/e (relative intensity) M⁺, 418, 416 (3.4, 7.2), 375 (8.4), 373 (21.8), 296 (6.8), 294 (6.8), 294 (16.0), 279 (7.0), 277 (18.5), 268 (42.7), 266 (100), 111 (65.4). Anal. calcd for C₁₅H₁₀ClFN₂O₅S₂ (416.85): C, 43.22; H, 2.42; N, 6.72. Found: C, 43.37; H, 2.30; N, 6.72.

### EXAMPLE 3

Using the indicated procedure and starting with the requisite reagents, the following compounds were prepared:

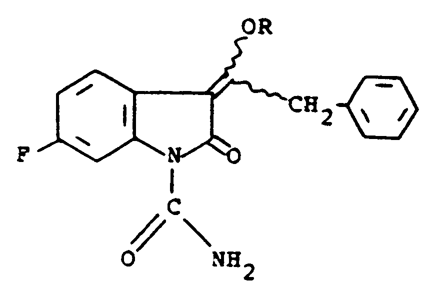

### Esters:

(R = -COCH₃) - Method A; yield 56% after recrystallization from 2-propanol; mp 195-197°C; mass spectrum m/e (relative intensity) M⁺, 354 (< 1), 312 (32.5), 269 (38.6), 251 (4.8), 221 (12.2), 194 (1.6), 178 (100), 121 (11.1), 91 (23.9), 65 (5.9); ¹H-NMR (d₆-Me₂SO) delta 2.31 (3H, s), 4.51 (2H, s), 7.02 (1H, dt, J=2.6, 8.9Hz), 7.33 (6H, s), 7.63 (1H, dd, J=5.8, 8.6Hz), 7.95 (2H, m). Anal. calcd for C₁₉H₁₅FN₂O₄ (354.19): C, 64.40; H, 4.27; N, 7.91. Found: C, 64.30; H, 4.21; N, 7.89.

(R = -COCH₂CH₃) - Method A; yield 23% after recrystallization from 2-propanol; mp 196-198°C; mass spectrum m/e (relative intensity) M⁺, 368 (2), 325 (5), 312 (25), 269 (70), 251 (7), 240 (4), 221 (5), 178 (100), 150 (8), 121 (10), 91 (37), 65 (12), 57 (83); ¹H-NMR (d₆-Me₂SO) delta 1.02 (3H, t, J=7.4Hz), 2.61 (2H, q, J=7.4Hz), 4.53 (2H, s), 7.02 (1H, dt, J=2.6, 9.2Hz), 7.32 (6H, m), 7.61 (1H, dd, J=5.8, 8.6Hz), 7.95 (2H, m). Anal. calcd for C₂₀H₁₇FN₂O₄ (358.20): C, 65.21; H, 4.65; N, 7.61. Found: C, 64.98; H, 4.44; N, 7.54.

(R = -COCH(CH₃)₂) - Method A; yield 28% after recrystallization from 2-propanol; mp 182-184°C; mass spectrum m/e (relative intensity) M⁺, 382 (3.5), 339 (< 1), 312 (18.6), 269 (18.1), 178 (46.2), 177 (17.4), 91 (31.8), 71 (100); ¹H-NMR (d₆-Me₂SO) delta 1.09 (3H, d, J=7.0Hz), 2.64 (1H, dq, J=7.0Hz), 4.65 (2H, s), 5.36 (1H, br s), 6.83 (1H, dt, J=2.5, 8.7Hz), 7.18-7.33 (5H, m), 7.50 (1H, dd, J=5.6, 8.6Hz), 8.10 (1H, dd, J=2.5, 10.3Hz), 8.59 (1H, br s). Anal. calcd for C₂₁H₁₉FN₂O₄ (382.38): C, 65.96; H, 5.01; N, 7.33. Found: C, 65.76; H, 4.94; N, 7.33.

(R = -COPh) - Method A; yield 68% after recrystallization from 2-propanol; mp 188-190°C; mass spectrum m/e (relative intensity) M⁺, 416 (2.7), 373 (3.0), 242 (6.1), 177 (6.4), 121 (5.2), 105 (100), 77 (17.8); ¹H-NMR (CDCl₃) delta 4.71 (2H, d), 5.41 (1H, br s), 6.71 (1H, dt, J=2.5, 8.7Hz), 7.26 (5H, m), 7.42 (1H, dd, J=5.6, 8.6), 7.52 (2H, m), 7.66 (1H, m), 8.03 (2H, d), 8.10 (1H, dd, J=2.5, 10.3Hz), 8.63 (1H, br s). Anal. calcd for C₂₄H₁₇FN₂O₄·H₂O (434.41): C, 66.35; H, 4.40; N, 6.44. Found: C, 66.14; H, 3.92; N, 6.41.

(R = -COCH₂Ph) - Method A; yield 27% after recrystallization from 2-propanol; mp 201-202°C; mass spectrum m/e (relative intensity) M⁺, 430 (0.9), 387 (0.6), 312 (87.5), 269 (100), 178 (64.7), 91 (65.6); ¹H-NMR (d₆-Me₂SO) delta 3.99 (2H, s), 4.48 (2H, s), 6.85 (2H, dt, J=2.6, 8.9Hz), 7.28 (10H, m), 7.91 (1H, dd, J=2.5, 10.7Hz), 7.97 (1H, br s), 8.07 (1H, br s). Anal. calcd for C₂₅H₁₉FN₂O₄ (430.25): C, 69.76; H, 4.45; N, 6.51. Found: C, 69.35; H, 4.38; N, 6.62.

(R = -COCH₂CH₂COOEt) - Method A; yield 46% after recrystallization from 2-propanol; mp 159-161°C; mass spectrum m/e (relative intensity) M⁺, not observed, 395 (0.4), 352 (0.8), 331 (0.3), 289 (0.6), 269 (6.4), 252 (9.4), 234 (1.9), 222 (6.8), 212 (1.5), 196 (1.1). 178 (24.8), 177 (10.6), 168 (1.5), 130 (7.7), 129 (100), 121 (5.3), 101 (65.8), 91 (10.0); ¹H-NMR (d₆-Me₂SO) delta 1.15 (3H, t, J=7.1Hz), 2.61 (2H, t, J=6.0Hz), 2.88 (2H, t, J=6.0Hz), 4.06 (2H, q, J=7.1Hz), 4.46 (2H, s), 6.98 (1H, dt, J=2.6, 8.9Hz), 7.32 (5H, m), 7.67 (1H, dd, J=5.8, 8.6Hz), 7.93 (1H, dd, J=2.5, 10.7Hz), 7.98 (1H, br s), 8.08 (1H, br s). Anal. calcd for C₂₃H₂₁FN₂O₆ (440.23): C, 62.72; H, 4.81; N, 6.36. Found: C, 62.75; H, 4.79; N, 6.29.

### Carbonates:

(R = -COOCH₃) - Method A; yield 45% after recrystallization from 2-propanol; mp 178-180°C; mass spectrum m/e (relative intensity) M⁺, 370 (16.7), 327 (3.7), 294 (24.0), 251 (100), 235 (11.7), 222 (32.9), 205 (0.4), 204 (3.8), 192 (29.8), 178 (42.5), 164 (2.2), 149 (5.8), ¹H-NMR (d₆-Me₂SO) delta 3.86 (3H, s), 4.58 (2H, s), 7.08 (1H, dt, J=2.6, 9.1Hz), 7.32 (5H, s), 7.55 (1H, dd, J=5.9, 8.7Hz), 7.95 (1H, dd, J=2.5, 10.6Hz), 7.99 (1H, br s), 8.07 (1H, br s). Anal. calcd for C₁₉H₁₅FN₂O₅ (370.19): C, 61.62; H, 4.08; N, 7.56. Found: C, 61.64; H, 4.07; N, 7.55.

(R = -COOCH₂CH₃) - Method A; yield 52% after recrystallization from 2-propanol; mp 189-190°C; mass spectrum m/e (relative intensity) M⁺, 384 (8.8), 340 (3.4), 312 (33.2), 297 (57.0), 269 (71.1), 251 (47.1), 240 (14.9), 221 (33.6), 212 (7.1), 206 (10.1), 178 (100), 150 (10.6), 121 (14.3), 91 (51.5); ¹H-NMR (d₆-Me₂SO) delta 1.23 (3H, t, J=7.1Hz), 4.26 (2H, q, J=7.1Hz), 4.58 (2H, s), 7.08 (1H, dt, J=2.5, 8.9Hz), 7.32 (5H, m), 7.53 (1H, dd, J=5.8, 8.6Hz), 7.94 (1H, dd, J=2.6, 10.7Hz), 7.99 (1H, br s), 8.07 (1H, br s). Anal. calcd for C₂₀H₁₇FN₂O₅ (384.19): C, 62.52; H, 4.42; N, 7.29. Found: C, 62.59; H, 4.41; N, 6.98.

(R = -COO(CH₂)₅CH₃) - Method A; yield 31% after recrystallization from 2-propanol; mp 144-145°C; mass spectrum m/e (relative intensity) M⁺, 440 (< 1), 397 (0.6), 378 (< 1), 353 (< 1), 312 (18.1), 294 (1.9), 269 (69.7), 251 (16.7), 240 (4.3), 221 (16.4), 212 (2.1), 194 (2.0), 178 (100), 164 (0.9), 149 (5.7), 121 (9.2), 103 (1.1), 91 (29.2); ¹H-NMR (d₆-Me₂SO) delta 0.85 (3H, br t,J=6.6Hz), 1.24 (6H, m), 1.58 (2H, m), 4.21 (2H, t, J=6.4Hz), 4.59 (2H, s), 7.06 (1H, dt, J=2.5, 9.0Hz), 7.31 (5H, m), 7.54 (1H, dd, J=5.8, 8.6Hz), 7.96 (1H, dd, J=2.5, 10.6Hz), 8.00 (1H, br s), 8.07 (1H, br s). Anal. calcd for C₂₄H₂₅FN₂O₅(440.24): C, 65.44; H, 5.72; N, 6.36. Found: C, 65.31; H, 5.62; N, 6.38.

## Claims (Claims for the following Contracting State(s): AT, BE, CH, DE, FR, GB, IT, LI, LU, NL, SE)

1. A compound of the formula 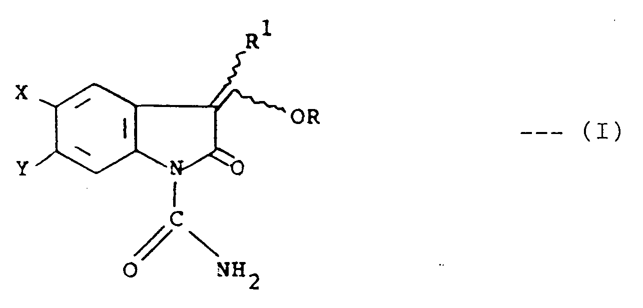 wherein X and Y are each selected from the group consisting of hydrogen, fluoro and chloro; R¹ is selected from the group consisting of 2-thienyl and benzyl; and R is selected from the group consisting of alkanoyl having two to ten carbon atoms, cycloalkylcarbonyl having five to seven carbon atoms, phenylalkanoyl having seven to ten carbon atoms, chlorobenzoyl, methoxybenzoyl, thenoyl, omega-alkoxycarbonylalkanoyl said alkoxy having one to three carbon atoms and said alkanoyl having three to five carbon atoms, alkoxycarbonyl having two to ten carbon atoms, phenoxycarbonyl, 1-(acyloxy)alkyl said acyl having one to four carbon atoms and said alkyl having one to four carbon atoms, 1-(alkoxycarbonyloxy)alkyl said alkoxy having two to five carbon atoms and said alkyl having one to four carbon atoms; alkyl of one to three carbon atoms; alkylsulfonyl having one to three carbon atoms, methylphenylsulfonyl and dialkylphosphonate said alkyl each having from one to three carbon atoms.

2. A compound of claim 1, wherein R¹ is 2-thienyl, X is chloro and Y is hydrogen.

3. A compound of claim 2, wherein R is alkanoyl having two to ten carbon atoms.

4. The compound of claim 3, wherein R is acetyl, isobutyryl or propionyl.

5. A compound of claim 2, wherein R is phenylalkanoyl having seven to ten carbon atoms.

6. The compound of claim 5, wherein R is phenylacetyl.

7. A compound of claim 2, wherein R is omega-alkoxycarbonylalkanoyl said alkoxy having one to three carbon atoms and said alkanoyl having three to five carbon atoms.

8. The compound of claim 7, wherein R is omega-ethoxycarbonylpropionyl.

9. A compound of claim 2, wherein R is alkoxycarbonyl having two to ten carbon atoms.

10. The compound of claim 9, wherein R is methoxycarbonyl, ethoxycarbonyl or n-hexoxycarbonyl.

11. A compound of claim 2, wherein R is 1-(alkoxycarbonyloxy)alkyl said alkoxy having two to five carbon atoms and said alkyl having one to four carbon atoms.

12. The compound of claim 11, wherein R is 1-(ethoxycarbonyloxy)ethyl.

13. A compound of claim 2, wherein R is alkylsulfonyl having one to three carbon atoms.

14. The compound of claim 13, wherein R is methylsulfonyl.

15. A compound of claim 1, wherein R¹ is 2-thienyl, X is fluoro and Y is chloro.

16. A compound of claim 15, wherein R is alkanoyl having two to ten carbon atoms.

17. A compound of claim 16, wherein R is acetyl, isobutyryl or propionyl.

18. A compound of claim 15, wherein R is alkoxycarbonyl having two to ten carbon atoms.

19. The compound of claim 18, wherien R is methoxycarbonyl, ethoxycarbonyl or n-hexoxycarbonyl.

20. A compound of claim 1, wherein R¹ is benzyl, X is hydrogen and Y is fluoro.

21. A compound of claim 20, wherein R is alkanoyl having two to ten carbon atoms.

22. The compound of claim 21, wherein R is acetyl.

23. A compound of claim 20, wherein R is alkoxycarbonyl having two to ten carbon atoms.

24. The compound of claim 23, wherein R is methoxycarbonyl.

25. A pharmaceutical composition comprising a compound of the formula (I) as claimed in any one of the preceding claims and a pharmaceutically acceptable diluent or carrier.

26. A compound of the formula (I) as claimed in any one of claims 1 to 24, for use as a medicament.

27. The use of a compound of the formula (I) as claimed in any one of claims 1 to 24 for the manufacture of a medicament for use as an antiinflammatory agent.

## Claims (Claims for the following Contracting State(s): ES, GR)

1. A process for preparing a compound of the formula 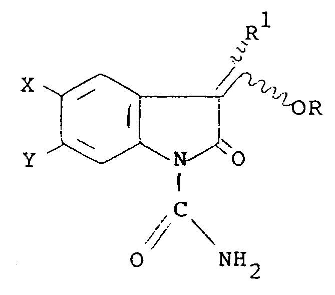 wherein X and Y are each selected from the group consisting of hydrogen, fluoro and chloro; R¹ is selected from the group consisting of 2-thienyl and benzyl; and R is selected from the group consisting of alkanoyl having two to ten carbon atoms, cycloalkylcarbonyl having five to seven carbon atoms, phenylalkanoyl having seven to ten carbon atoms, chlorobenzoyl, methoxybenzoyl, thenoyl, omega-alkoxycarbonylalkanoyl said alkoxy having one to three carbon atoms and said alkanoyl having three to five carbon atoms, alkoxycarbonyl having two to ten carbon atoms, phenoxycarbonyl, 1-(acyloxy)alkyl said acyl having one to four carbon atoms and said alkyl having one to four carbon atoms, alkyl of one to three carbon atoms, alkylsulfonyl having one to three carbon atoms, methylphenylsulfonyl and dialkylphosphonate said alkyl each having from one to three carbon atoms which comprises reacting a compound of the formula 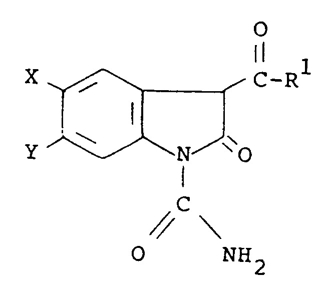 with an equimolar amount or slight excess of the requisite acid chloride, chloroformate, oxonium salt or alkylating agent in a reaction-inert solvent containing an equimolar amount of a tertiary amine at 0°C until the reaction is substantially complete.

2. The process of claim 1, wherein the solvent is chloroform and the tertiary amine is triethylamine.

3. A process for preparing a compound of the formula 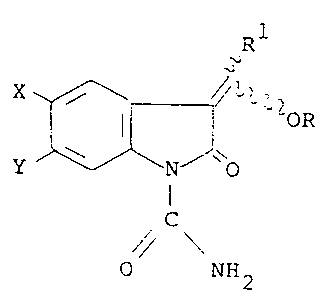 wherein X and Y are each selected from the group consisting of hydrogen, fluoro and chloro; R¹ is selected from the group consisting of 2-thienyl and benzyl; and R is 1-(alkoxycarbonyloxy)alkyl said alkoxy having two to five carbon atoms and said alkyl having one to four carbon atoms which comprises reacting a compound of the formula 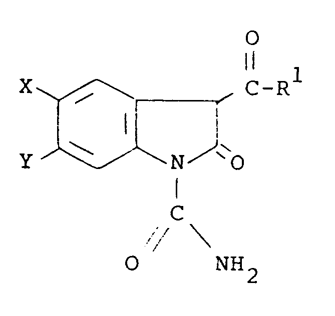 with about a 3-4 fold molar excess of the requisite alpha-chloroalkylcarbonate in a reaction-inert solvent containing about a five fold molar excess of sodium iodide and about a two fold molar excess of an alkali metal carbonate until the reaction is substantially complete.

4. The process of claim 3, wherein the reaction-inert solvent is acetone and the alkali metal carbonate is potassium carbonate.

## Patentansprüche (Patentansprüche für folgende(n) Vertragsstaat(en): AT, BE, CH, DE, FR, GB, IT, LI, LU, NL, SE)

1. Verbindung der Formel 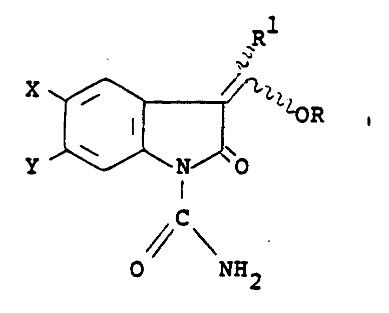 worin X und X jeweils ausgewählt sich aus der aus Wasserstoff, Fluor und Chlor bestehenden Gruppe; R¹ ausgewählt ist aus der aus 2-Thienyl und Benzyl bestehenden Gruppe; und R ausgewählt ist aus der aus Alkanoyl mit zwei bis zehn Kohlenstoffatomen, Cycloalkylcarbonyl mit fünf bis sieben Kohlenstoffatomen, Phenylalkanoyl mit sieben bis zehn Kohlenstoffatomen, Chlorbenzoyl, Methoxybenzoyl, Thenoyl, omega-Alkoxycarbonylalkanoyl, wobei das Alkoxy ein bis drei Kohlenstoffatome und das Alkanoyl drei bis fünf Kohlenstoffatome hat, Alkoxycarbonyl mit zwei bis zehn Kohlenstoffatomen, Phenoxycarbony, 1-(Acyloxy)alkyl, wobei das Acyl ein bis vier Kohlenstoffatome und das Alkyl ein bis vier Kohlenstoffatome hat, 1-(Alkoxycarbonyloxy)alkyl, wobei das Alkoxy zwei bis fünf Kohlenstoffatome und das Alkyl ein bis vier Kohlenstoffatome hat; Alkyl mit ein bis drei Kohlenstoffatomen, Alkylsulfonyl mit ein bis drei Kohlenstoffatomen, Methylphenylsulfonyl und Dialkylphosphonat, wobei das Alkyl jeweils ein bis drei Kohlenstoffatome hat, bestehenden Gruppe.

2. Verbindung nach Anspruch 1, worin R¹ 2-Thienyl ist, X Chlor ist und Y Wasserstoff ist.

3. Verbindung nach Anspruch 2, worin R Alkanoyl mit zwei bis zehn Kohlenstoffatomen ist.

4. Verbindung nach Anspruch 3, worin R Acetyl, Isobutyryl oder Propionyl ist.

5. Verbindung nach Anspruch 2, worin R Phenylalkanoyl mit sieben bis zehn Kohlenstoffatomen ist.

6. Verbindung nach Anspruch 5, worin R Phenylacetyl ist.

7. Verbindung nach Anspruch 2, worin R omega-Alkoxycarbonylalkanoyl ist, wobei das Alkoxy ein bis drei Kohlenstoffatome hat und das Alkanoyl drei bis fünf Kohlenstoffatome hat.

8. Verbindung nach Anspruch 7, worin R omega-Ethoxycarbonylpropionyl ist.

9. Verbindung nach Anspruch 2, worin R Alkoxycarbonyl mit zwei bis zehn Kohlenstoffatomen ist.

10. Verbindung nach Anspruch 9, worin R Methoxycarbonyl, Ethoxycarbonyl oder n-Hexoxycarbonyl ist.

11. Verbindung nach Anspruch 2, worin R 1-(Alkoxycarbonyloxy)alkyl ist, wobei das Alkoxy zwei bis fünf Kohlenstoffatome hat und das Alkyl ein bis vier Kohlenstoffatome hat.

12. Verbindung nach Anspruch 11, worin R 1-(Ethoxycarbonyloxy)ethyl ist.

13. Verbindung nach Anspruch 2, worin R Alkylsulfonyl mit ein bis drei Kohlenstoffatomen ist.

14. Verbindung nach Anspruch 13, worin R Methylsulfonyl ist.

15. Verbindung nach Anspruch 1, worin R¹ 2-Thienyl ist, X Fluor ist und Y Chlor ist.

16. Verbindung nach Anspruch 15, worin R Alkanoyl mit zwei bis zehn Kohlenstoffatomen ist.

17. Verbindung nach Anspruch 16, worin R Acetyl, Isobutyryl oder Propionyl ist.

18. Verbindung nach Anspruch 15, worin R Alkoxycarbonyl mit zwei bis zehn Kohlenstoffatomen ist.

19. Verbindung nach Anspruch 18, worin R Methoxycarbonyl, Ethoxycarbonyl oder n-Hexoxycarbonyl ist.

20. Verbindung nach Anspruch 1, worin R¹ Benzyl ist, X Wasserstoff ist und Y Fluor ist.

21. Verbindung nach Anspruch 20, worin R Alkanoyl mit zwei bis zehn Kohlenstoffatomen ist.

22. Verbindung nach Anspruch 21, wobein R Acetyl ist.

23. Verbindung nach Anspruch 20, worin R Alkoxycarbonyl mit zwei bis zehn Kohlenstoffatomen ist.

24. Verbindung nach Anspruch 23, worin R Methoxycarbonyl ist.

25. Pharmazeutische Zusammensetzung, umfassend eine Verbindung der Formel (I) nach einem der vorhergehenden Ansprüche und ein pharmazeutisch annehmbares Verdünnungsmittel oder einen solchen Träger.

26. Verbindung der Formel (I) nach einem der Ansprüche 1 bis 24 zur Verwendung als Arzneimittel.

27. Verwendung einer Verbindung der Formel (I) nach einem der Ansprüche 1 bis 24 zur Herstellung eines Arzneimittels für die Verwendung als entzündungshemmendes Mittel.

## Patentansprüche (Patentansprüche für folgende(n) Vertragsstaat(en): ES, GR)

1. Verfahren zur Herstellung einer Verbindung der Formel 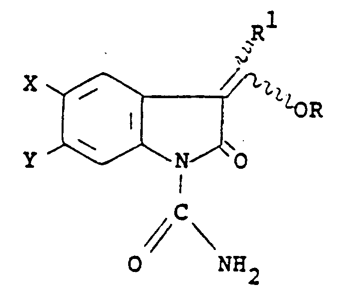 worin X und X jeweils ausgewählt sich aus der aus Wasserstoff, Fluor und Chlor bestehenden Gruppe; R¹ ausgewählt ist aus der aus 2-Thienyl und Benzyl bestehenden Gruppe; und R ausgewählt ist aus der aus Alkanoyl mit zwei bis zehn Kohlenstoffatomen, Cycloalkylcarbonyl mit fünf bis sieben Kohlenstoffatomen, Phenylalkanoyl mit sieben bis zehn Kohlenstoffatomen, Chlorbenzoyl, Methoxybenzoyl, Thenoyl, omega-Alkoxycarbonylalkanoyl, wobei das Alkoxy ein bis drei Kohlenstoffatome und das Alkanoyl drei bis fünf Kohlenstoffatome hat, Alkoxycarbonyl mit zwei bis zehn Kohlenstoffatomen, Phenoxycarbony, 1-(Acyloxy)alkyl, wobei das Acyl ein bis vier Kohlenstoffatome und das Alkyl ein bis vier Kohlenstoffatome hat, 1-(Alkoxycarbonyloxy)alkyl, wobei das Alkoxy zwei bis fünf Kohlenstoffatome und das Alkyl ein bis vier Kohlenstoffatome hat; Alkyl mit ein bis drei Kohlenstoffatomen, Alkylsulfonyl mit ein bis drei Kohlenstoffatomen, Methylphenylsulfonyl und Dialkylphosphonat, wobei das Alkyl jeweils ein bis drei Kohlenstoffatome hat, bestehenden Gruppe,
das umfaßt das Umsetzen einer Verbindung der Formel 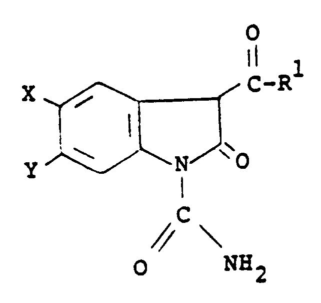 mit einer äquimolaren Menge oder einem leichten Überschuß des erforderlichen Säurechlorids, Chlorformiats, Oxoniumsalzes oder Alkylierungsmittels in einem reaktionsinerten Lösungsmittel, das eine äquimolare Menge eines tertiären Amins enthält, bei 0°C, bis die Reaktion im wesentlichen vollständig ist.

2. Verfahren nach Anspruch 1, bei dem das Lösungsmittel Chloroform ist und das tertiäre Amin Triethylamin ist.

3. Verfahren zur Herstellung einer Verbindung der Formel 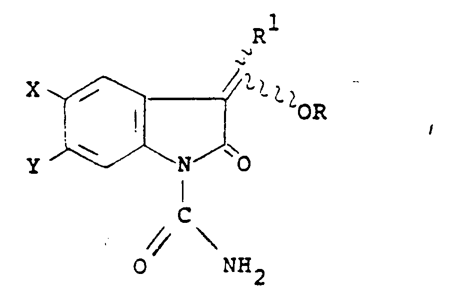 worin X und Y jeweils ausgewählt sind auch der aus Wasserstoff, Fluor und Chlor bestehenden Gruppe; R¹ ausgewählt ist aus der aus 2-Thienyl und Benzyl bestehenden Gruppe; und R 1-(Alkoxycarbonyloxy)alkyl ist, wobei das Alkoxy zwei bis fünf Kohlenstoffatome und das Alkyl ein bis vier Kohlenstoffatome hat, das umfaßt das Umsetzen einer Verbindung der Formel 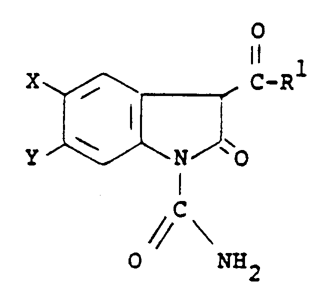 mit einem etwa drei- bis vierfachen molaren Überschuß des erforderlichen alpha-Chloralkylcarbonates in einem reaktionsinerten Lösungsmittel, das etwa einen fünffachen molaren Überschuß Natriumjodid und etwa einen zweifachen molaren Überschuß eines Alkalimetallcarbonates enthält, bis die Reaktion im wesentlichen vollständig ist.

4. Verfahren nach Anspruch 3, bei dem das reaktionsinerte Lösungsmittel Aceton ist und das Alkalimetallcarbonat Kaliumcarbonat ist.

## Revendications (Revendications pour l'(les) Etat(s) contractant(s) suivant(s): AT, BE, CH, DE, FR, GB, IT, LI, LU, NL, SE)

1. Composé de formule 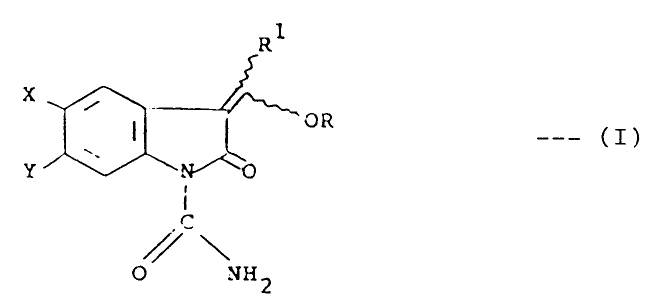 dans laquelle X et Y sont choisis chacun dans le groupe consistant en l'hydrogène, un groupe fluoro et un groupe chloro ; R¹ est choisi dans le groupe consistant en un groupe 2-thiényle et un groupe benzyle ; et R est choisi dans le groupe consistant en des groupes alcanoyle ayant deux à dix atomes de carbone, cycloalkylcarbonyle ayant cinq à sept atomes de carbone, phénylalcanoyle ayant sept à dix atomes de carbone, chlorobenzoyle, méthoxybenzoyle, thénoyle, oméga-alkoxycarbonylalcanoyle dont le groupe alkoxy possède un à trois atomes de carbone et le groupe alcanoyle possède trois à cinq atomes de carbone, alkoxycarbonyle ayant deux à dix atomes de carbone, phénoxycarbonyle, 1-(acyloxy)alkyle dont le groupe acyle possède un à quatre atomes de carbone et le groupe alkyle possède un à quatre atomes de carbone, 1-(alkoxycarbonyloxy)alkyle dont le groupe alkoxy possède deux à cinq atomes de carbone et le groupe alkyle possède un à quatre atomes de carbone ; alkyle ayant un à trois atomes de carbone, alkylsulfonyle ayant un à trois atomes de carbone, méthylphénylsulfonyle et dialkylphosphonate dont chaque groupe alkyle possède un à trois atomes de carbone.

2. Composé suivant la revendication 1, dans lequel R¹ représente un groupe 2-thiényle, X représente un groupe chloro et Y représente l'hydrogène.

3. Composé suivant la revendication 2, dans lequel R représente un groupe alcanoyle ayant deux à dix atomes de carbone.

4. Composé suivant la revendication 3, dans lequel R représente un groupe acétyle, isobutyryle ou propionyle.

5. Composé suivant la revendication 2, dans lequel R représente un groupe phénylalcanoyle ayant sept à dix atomes de carbone.

6. Composé suivant la revendication 5, dans lequel R représente un groupe phénylacétyle.

7. Composé suivant la revendication 2, dans lequel R représente un groupe oméga-alkoxycarbonylalcanoyle dont le groupe alkoxy possède un à trois atomes de carbone et le groupe alcanoyle possède trois à cinq atomes de carbone.

8. Composé suivant la revendication 7, dans lequel R représente un groupe oméga-éthoxycarbonylpropionyle.

9. Composé suivant la revendication 2, dans lequel R représente un groupe alkoxycarbonyle ayant deux à dix atomes de carbone.

10. Composé suivant la revendication 9, dans lequel R représente un groupe méthoxycarbonyle, éthoxycarbonyle ou n-hexoxycarbonyle.

11. Composé suivant la revendication 2, dans lequel R représente un groupe 1-(alkoxycarbonyloxy)alkyle dont le groupe alkoxy possède deux à cinq atomes de carbone et le groupe alkyle possède un à quatre atomes de carbone.

12. Composé suivant la revendication 11, dans lequel R représente un groupe 1-(éthoxycarbonyloxy)éthyle.

13. Composé suivant la revendication 2, dans lequel R représente un groupe alkylsulfonyle ayant un à trois atomes de carbone.

14. Composé suivant la revendication 13, dans lequel R représente un groupe méthylsulfonyle.

15. Composé suivant la revendication 1, dans lequel R¹ représente un groupe 2-thiényle, X représente un groupe fluoro et Y représente un groupe chloro.

16. Composé suivant la revendication 15, dans lequel R représente un groupe alcanoyle ayant deux à dix atomes de carbone.

17. Composé suivant la revendication 16, dans lequel R représente un groupe acétyle, isobutyryle ou propionyle.

18. Composé suivant la revendication 15, dans lequel R représente un groupe alkoxycarbonyle ayant deux à dix atomes de carbone.

19. Composé suivant la revendication 18, dans lequel R représente un groupe méthoxycarbonyle, éthoxycarbonyle ou n-hexoxycarbonyle.

20. Composé suivant la revendication 1, dans lequel R¹ représente un groupe benzyle, X représente l'hydrogène et Y représente un groupe fluoro.

21. Composé suivant la revendication 20, dans lequel R représente un groupe alcanoyle ayant deux à dix atomes de carbone.

22. Composé suivant la revendication 21, dans lequel R représente un groupe acétyle.

23. Composé suivant la revendication 20, dans lequel R représente un groupe alkoxycarbonyle ayant deux à dix atomes de carbone.

24. Composé suivant la revendication 23, dans lequel R représente un groupe méthoxycarbonyle.

25. Composition pharmaceutique comprenant un composé de formule (I) suivant l'une quelconque des revendications précédentes et un diluant ou support pharmaceutiquement acceptable.

26. Composé de formule (I) suivant l'une quelconque des revendications 1 à 24, destiné à être utilisé comme médicament.

27. Utilisation d'un composé de formule (I) suivant l'une quelconque des revendications 1 à 24 pour la production d'un médicament destiné à être utilisé comme agent anti-inflammatoire.

## Revendications (Revendications pour l'(les) Etat(s) contractant(s) suivant(s): ES, GR)

1. Procédé de préparation d'un composé de formule 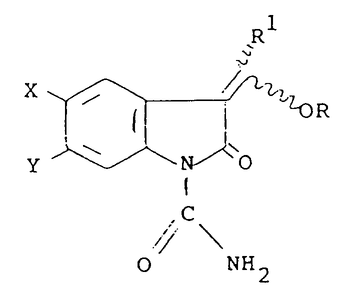 dans laquelle X et Y sont choisis chacun dans le groupe consistant en l'hydrogène, un groupe fluoro et un groupe chloro ; R¹ est choisi dans le groupe consistant en un groupe 2-thiényle et un groupe benzyle ; et R est choisi dans le groupe consistant en des groupes alcanoyle ayant deux à dix atomes de carbone, cycloalkylcarbonyle ayant cinq à sept atomes de carbone, phénylalcanoyle ayant sept à dix atomes de carbone, chlorobenzoyle, méthoxybenzoyle, thénoyle, oméga-alkoxycarbonylalcanoyle dont le groupe alkoxy possède un à trois atomes de carbone et le groupe alcanoyle possède trois à cinq atomes de carbone, alkoxycarbonyle ayant deux à dix atomes de carbone, phénoxycarbonyle, 1-(acyloxy)alkyle dont le groupe acyle possède un à quatre atomes de carbone et le groupe alkyle possède un à quatre atomes de carbone, alkyle ayant un à trois atomes de carbone, alkylsulfonyle ayant un à trois atomes de carbone, méthylphénylsulfonyle et dialkylphosphonate dont chaque groupe alkyle possède un à trois atomes de carbone, qui comprend la réaction d'un composé de formule 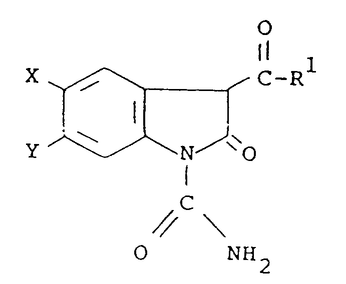 avec une quantité équimolaire ou un léger excès du chlorure d'acide, chloroformiate, sel d'oxonium ou agent d'alkylation requis dans un solvant inerte vis-à-vis de la réaction, contenant une quantité équimolaire d'une amine tertiaire, à 0°C jusqu'à ce que la réaction soit parvenue pratiquement à son terme.

2. Procédé suivant la revendication 1, dans lequel le solvant est le chloroforme et l'amine tertiaire est la triéthylamine.

3. Procédé de préparation d'un composé de formule 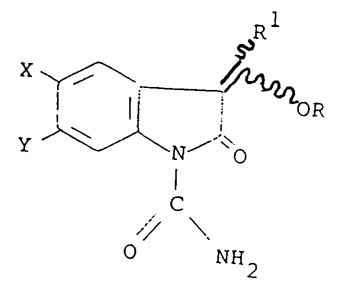 dans laquelle X et Y sont choisis chacun dans le groupe consistant en l'hydrogène, un groupe fluoro et un groupe chloro ; R¹ est choisi dans le groupe consistant en un groupe 2-thiényle et un groupe benzyle ; et R représente un groupe 1-(alkoxycarbonyloxy)alkyle dont le groupe alkoxy possède deux à cinq atomes de carbone et le groupe alkyle possède un à quatre atomes de carbone, qui comprend la réaction d'un composé de formule 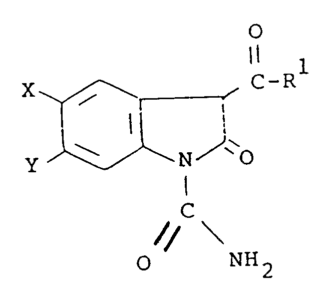 avec un excès molaire approximativement triple à quadruple de l'alpha-chloroalkylcarbonate requis dans un solvant inerte vis-à-vis de la réaction contenant un excès molaire approximativement quintuple d'iodure de sodium et un excès molaire approximativement double d'un carbonate de métal alcalin jusqu'à ce que la réaction soit pratiquement parvenue à son terme.

4. Procédé suivant la revendication 3, dans lequel le solvant inerte vis-à-vis de la réaction est l'acétone et le carbonate de métal alcalin est le carbonate de potassium.
